# EUROPEAN PATENT SPECIFICATION

(11) **EP 1 943 973 B1**
(45) Date of publication and mention of the grant of the patent: **07.04.2010**
(21) Application number: 08250153.7
(22) Date of filing: 11.01.2008
(51) Int. Cl.: A61B 18/14

(54) **Ablation system and clamp**
Ablationssystem und Klemme
Système d'ablation et pince

(30) Priority: 12.01.2007 US 884783 P; 12.01.2007 US 884719 P
(43) Date of publication of application: 16.07.2008
(73) Proprietor: AtriCure Inc., West Chester, OH 45069 (US)
(72) Inventor: Martin, Keith Edward, Mason, Ohio 45040 (US); Privitera, Salvatore, Mason, Ohio 45040 (US); Park, Christopher J., Oregonia, Ohio 45054 (US)
(74) Representative: Dee, Ian Mark

(56) References cited:
- US-A1- 2001 008 967
- US-A1- 2002 091 384
- US-A1- 2004 039 429
- US-A1- 2004 068 274
- US-A1- 2005 090 815

## Description

### Field of the Invention

The present invention relates, in general, to an apparatus for tissue ablation instrument and, more particularly, to devices and systems using resistive and thermal heating for the ablation of tissue.

### Background of the Invention

It is known to use bipolar RF energy devices that clamp tissue between opposed electrodes and apply RF energy to heat tissue to form lines of ablation on the tissue. This has found particular application in the formation of strategically located lines of ablation in cardiac tissue to block spurious electrical signals to the heart, which has been particularly beneficial in the treatment of atrial fibrillation. See, e.g., U.S. Patent No. 6,889,694.

Such bipolar electrode devices apply energy directly to the surface of tissue clamped between a first electrode and a second electrode. The first electrode, the clamped tissue, and the second electrode form a conductive resistive circuit. As the moisture in the tissue conducts the RF energy, the tissue begins to desiccate. As the tissue desiccates it becomes more resistive. The application of bipolar RF energy on tissue shows that tissue desiccation progresses inwardly from the outside or surface of the tissue near the electrode-tissue contact area, where the current flux or density is greatest. Surface desiccation increases resistance in the tissue and can make it more difficult to achieve good depth of penetration in underlying tissue without creating a larger than desired area of ablated tissue or excessive surface heating adjacent to the electrodes. A recent study reported that to achieve a depth of ablation of 5 mm, a width of ablation of almost 8 mm wide in the endocardium resulted. See, "Mechanism, Localization, and Cure of Atrial Arrhythmias Occurring After a New Intraoperative Endocardial Radiofrequency Ablation Procedure for Atrial Fibrillation," Thomas, et al., Journal of the American College of Cardiology, Vol. 35, No. 2, 2000. While a wider ablation facilitates the surgeon's visual confirmation that an ablation has been created, it is still desirable to control the width of ablation so as to keep the width of the ablation and thermal spread from the zone of ablation within certain limits so as to not irreversibly damage more cardiac tissue than necessary.

US 2005/0090815 discloses an ablation system for creating lesions and assessing their completeness or transmurality.

To overcome surface tissue heating effects, techniques such as cooling or cryogenics have been used, and selected positioning of the electrodes has been considered. See, for example, U.S. Patent Nos. 6,413,253 to Koop et al., 6,629,535 to Ingle et al., and 7,022,121 to Stern et al. and 6,918,906 to Long.

Consequently, a significant need exists for an improved electrosurgical device and method of use that can enhance lesion formation and provide for more efficient ablation.

### Brief Summary of the Invention

The invention provides a system for ablating tissue according to claim 1.

The system can be used in a method of tissue ablation in which the tissue to be ablated is contacted with a plurality of electrode pairs, the electrodes of each pair being of opposite RF energy polarity so as to provide a current flux between the members of each pair when activated. The electrode pairs are then alternately activated and deactivated with RF energy to create at least one zone of primary heating in the tissue that is spaced from or substantially non-coincident with at least one zone of the highest current flux in the tissue. This is unlike the prior art devices where the zones of primary heating and highest current density substantially coincide or contained in the same space and are located at the electrode-tissue interface.

More specifically, a tissue ablation apparatus is provided that comprises opposed relatively moveable jaws for clamping the tissue to be ablated therebetween. A plurality of electrode pairs is provided, one electrode of each pair being carried on one jaw and being adapted to be connected to one terminal of an RF generator, and the other electrode of each pair being carried on the other jaw and being adapted to be connected to the opposite terminal of an RF generator. A current flux is created between the respective electrodes of each pair when activated by the generator. The electrode pairs are located on the jaws such that when alternately activated and deactivated by and RF generator, the electrodes create at least one flux zone of primary heating in the tissue that is spaced from at least one flux zone of the highest current flux in the tissue.

In another embodiment of the invention, the device can be provided with multiple pairs of energy sources or electrodes. For example, if two pairs are provided, one zone of overlap results, if three pairs are provided, two zones of overlap result, if four pairs are provided, three zones of overlap result, etc. The number of pairs of energy sources can be selected to provide an ablation line, area or zone of the desired width.

When the system of the invention is used in a method of tissue ablation, sufficient pressure is preferably applied to the tissue to be ablated to reduce its moisture content, and also to normalize the impedance within the tissue and to provide good contact between the electrodes and the tissue to normalize the electrode to tissue impedance. Preferably, the pressure applied to the tissue is proportional to its thickness, i.e. increasing pressure with increasing tissue thickness. This pressure may be the result of clamping force, suction, or other means.

In another embodiment, the device may utilize impedance and/or temperature sensors to control the energy delivered to the instrument.

These and other aspects and advantages of the present invention will become apparent from the accompanying drawings and the following detailed description.

### Brief Description of the Drawings

The accompanying drawings, which are incorporated in and constitute a part of this specification, illustrate embodiments of the invention, and, together with the general description of the invention given above, and the detailed description of the embodiments given below, serve to explain the principles of the present invention.

Fig. 1 is a perspective view of a system according to the present invention including an RF energy generator and a cardiac ablation clamp having opposed jaws.

Fig. 2 is an enlarged fragmentary perspective view of the opposed jaws of the ablation clamp shown in Fig. 1.

Fig. 3 is a plan view of one of the jaws of the ablation clamp of Fig. 1.

Fig. 4 is an enlarged cross-sectional view of the opposed jaws of the RF ablation clamp of Fig. 1 taken along line 4-4 of Fig. 3.

Figs. 5-7 illustrate the creation of overlapping energy fields between two pairs of spaced apart electrodes by alternately energizing the electrodes of each pair in accordance with the present invention.

Figs. 8-10 sequentially illustrate the formation of a lesion in tissue held between opposed jaw members having two pairs of opposed electrodes in accordance with the present invention.

Figs. 11-13 illustrate the creation of overlapping energy fields when three pairs of opposed electrodes are used in accordance with the present invention.

Fig. 14 is a cross-sectional view of tissue comparing the profile of the ablated tissue when the system according to the present invention is used and that obtained by practicing the prior art.

Fig. 15 is a schematic diagram of a controller for use with the ablation instrument of the present application.

### Detailed Description of the Invention

The following description of certain examples of the invention is not intended and should not be used to limit the scope of the present invention. Other examples, features, aspects, embodiments, and advantages of the invention will become apparent to those skilled in the art from the following description, which is by way of illustration of one or more of the best modes contemplated for carrying out the invention. As will be realized, the invention is capable of other different and obvious aspects, all without departing from the invention. Accordingly, the drawings and descriptions should be regarded as illustrative in nature and not restrictive.

As described above, radio frequency (RF) energy can be used in electrosurgical systems for heating, coagulation, or ablating tissue. Monopolar and bipolar RF systems are known to those skilled in the art, and it is well known to use a bipolar electrosurgical clamping device. Bipolar electrosurgical instruments apply energy between a pair of electrodes in direct contact with the tissue to be ablated and provide more precise control of the extent of ablation than monopolar energy. It is well known to use a bipolar electrosurgical a clamping device with a pair of opposed RF electrodes to clamp and ablate tissue therebetween, and the ablated tissue may produce a generally continuous lesion which can be well suited for the treatment of cardiac aryhythmias. The Atricure Isolator® and Ablation and Sensing Unit ("ASU") from Atricure, Inc. of Cincinnati, Ohio, is a system useful for such treatment of atrial fibrillation.

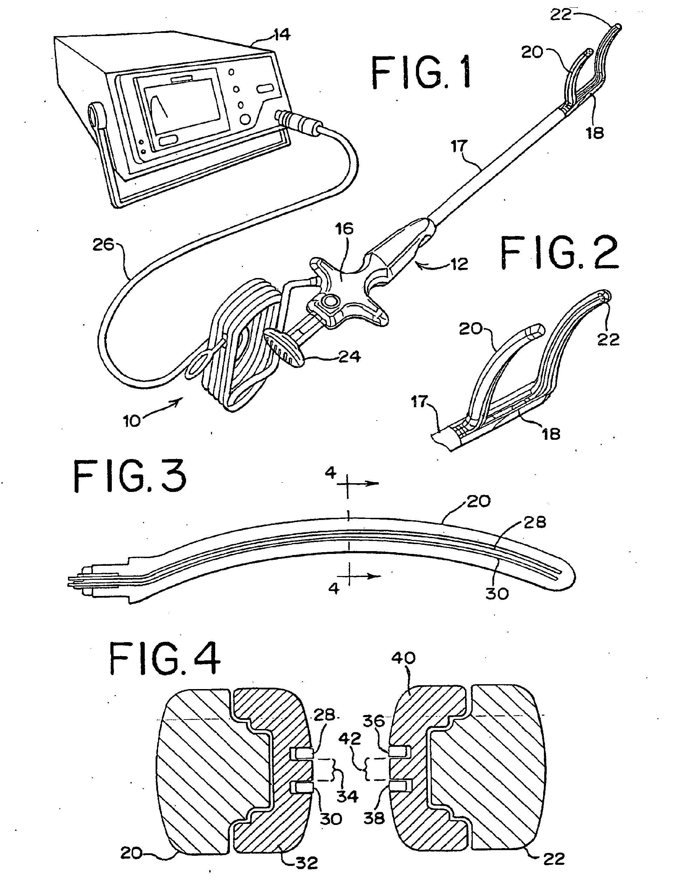

In accordance with one aspect of the present invention, FIG. 1 illustrates an example of a tissue ablation system in the form of a bipolar electrosurgical system 10 having an electrosurgical instrument 12 coupled to an energy generator, e.g., an RF generator 14. Electrosurgical instrument 10 preferably comprises a handle 16, an elongated longitudinal shaft 17 extending therefrom, and an end effector 18 for clamping and heating tissue therebetween. Although illustrated as a clamping device particularly suited for open procedures where the ablation site is directly viewable by the surgeon, the present invention is also well suited for minimally invasive procedures, such as intercostal or subxyphoid approaches to cardiac tissue targeted for ablation.

The illustrated end effector 18 has first and second opposed jaws for clamping tissue therebetween, henceforth referred to for convenience as proximal jaw 20 and distal jaw 22. The proximal and distal jaws 20, 22 are shown spaced apart for the reception of tissue therebetween, but at least one of the proximal and distal jaws 20, 22 respectively could be movable to clamp tissue therebetween. To this end, proximal and distal jaws 20, 22 may be operably coupled to a closure trigger 24 extending proximally from the handle 16 such that it is operable with one hand so that distal movement of closure trigger 24 brings the proximal and distal jaws 20, 22 together. Likewise, proximal movement of closure trigger 24 moves the proximal and distal jaws 20, 22 apart. The proximal and distal jaws 20, 22 are shown extending at an angle from the shaft 16, but can be at any angle with the shaft 17. The present invention is not limited to the particular mechanism for moving the jaw(s) and an example of such a mechanism may be found in U.S. Patent No. 6,923,806 and U.S. Patent No. 7,291,161.

The system of the invention can be used in a method for ablating tissue that comprises positioning two or more preferably bi-directional ablation energy sources in spaced-apart relation in sufficient proximity to the tissue to be ablated so that, upon activation each energy source creates an energy field in the tissue to be ablated. The energy sources are spaced such that the energy fields created by at least one of the activated sources partially overlaps with the energy field created by one or more of the other energy sources. The energy sources are alternately activated and deactivated, so that a substantially constant energy field results where the energy fields created by at least two of the energy sources overlap. While the energy sources are preferably RF energy sources, other energy sources, such as microwave, may be used.

To that end, and in keeping with one system of the invention that employs RF energy, two or more pairs of opposed electrodes are located in proximal and distal jaws 20, 22 of the instrument 12. All of the electrodes are operably coupled to the RF generator 14 by a cable 26 and can be operator actuated by a switch, such as a foot switch extending therefrom. A control system/RF generator for providing RF energy is generally shown in U.S. Patent Appln. Pub US 2007/0191826, published August 16, 2007. A controller for use in the system of the present invention is more specifically shown in the U.S. application Pub US 2008/0114350 filed January 7, 2007 in the name of Chris Park et al. entitled Matrix Router for Surgical Ablation.

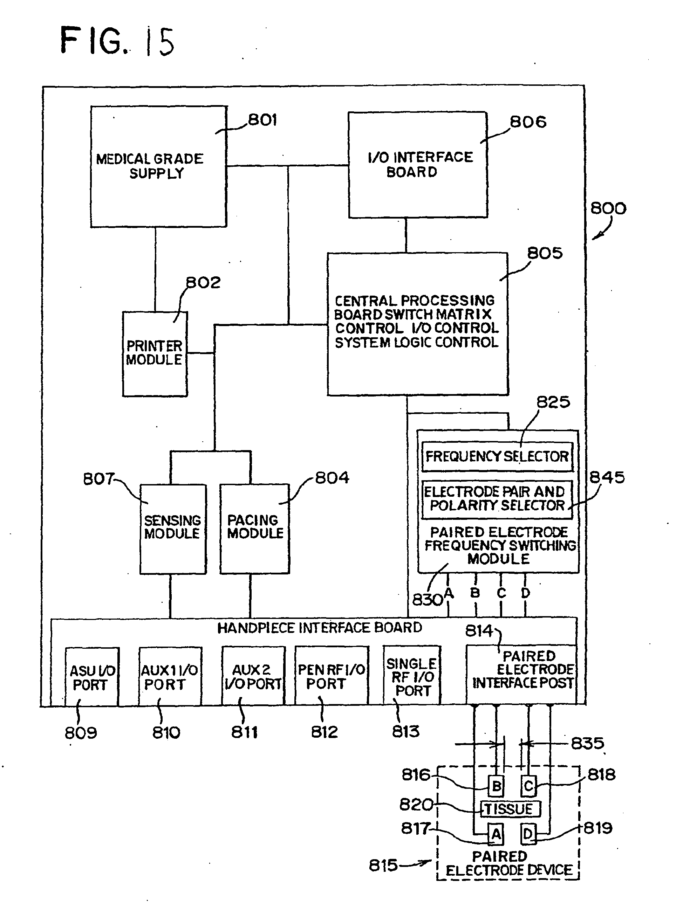

With reference to Fig. 15 a schematic a switching matrix router (800) for controlling the delivery of ablation energy or other signals to the ablation instrument is shown. The switching matrix router (800) can comprise an energy source (801) which can supply energy such as bipolar RF energy, a printer control module (802), a handpiece interface circuit (803), a pacing module (804), a control circuit (805), an input/output circuit (806), and a sensing module (807). In the illustrated embodiment, handpiece interface board (803) can comprise a plurality of I/O ports for the connection of surgical devices to the switching matrix router (800). The plurality of I/O ports can include an ASU port (809) for connection of the ASU, an AUX 1 I/O port (810) for connection of a first auxiliary device such as a sensing or pacing unit, an AUX 2 I/O port (811) for connection of a second auxiliary device such as a sensing or pacing unit, a pen RF I/O port (812) for connection to a RF pen, a single RF I/O port (813) for the connection to a surgical device having a single pair of opposed bipolar electrodes, and a twin RF I/O port or paired electrode interface port (814). The paired electrode interface port (814) could be provided for the connection of a paired electrode device (815) to the switching matrix router (800). The paired electrode device (815) can have at least two pairs of bipolar electrodes such as a first pair of opposed electrodes (816, 817) and a second pair of opposed electrodes (818, 819) shown opposed on opposite sides of a portion of tissue (820). The paired electrode device (815) can have one wire extending from each electrode (815, 816, 817, 818) to electrically connect the electrodes to the electrode interface port (814). The pairs of electrodes (816, 817) and (818, 819) may be opposably mounted in a clampable portion of the paired electrode device (815), such as a first jaw and a second jaw, and one or both of the jaws can be moved together to clamp tissue (820) between the jaws and electrodes (816, 817) and (818, 819). Energy can be applied to the clamped tissue 820 across the first electrode pair (816, 817) and across the second electrode pair (818, 819). The application of energy such as bipolar RF energy to the clamped tissue (820) can cauterize the clamped tissue.

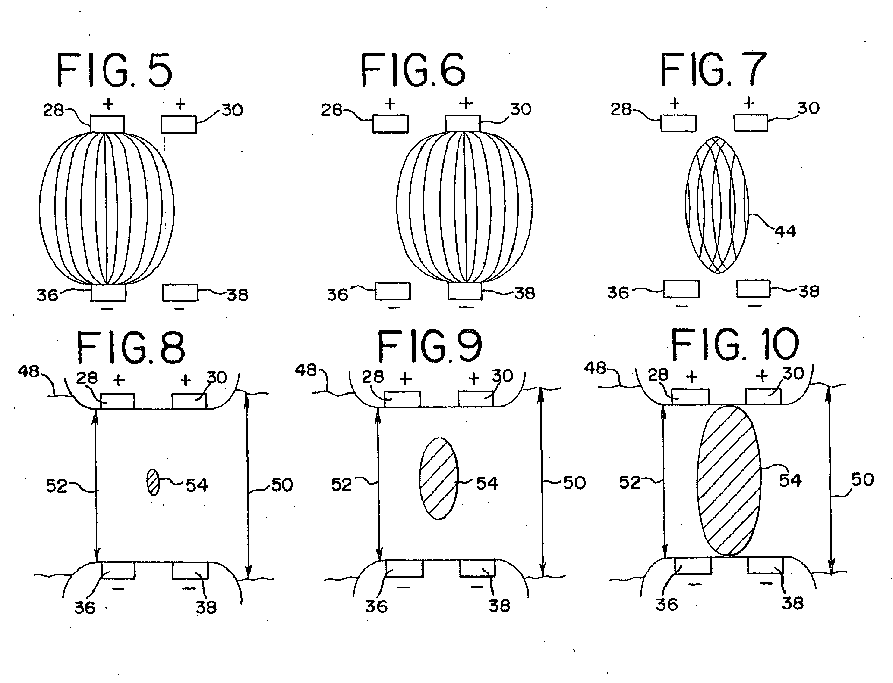

In Fig. 8, the paired electrode device (815) is shown as having the electrodes (816, 817, 818, 819) spaced on two sides of the tissue (820). The electrode pairs (816, 817) and (818, 819) can be spaced apart an amount such as space (835), and, by way of example, the electrode pairs could be located parallel to one another.

Whereas the above description describes the application of energy across the first pair of opposed electrodes (816, 817) and across the second pair of opposed electrodes (818, 819), one or more additional pairs of electrodes spaced from the first and second pairs may be provided, with energy being selectively applied to tissue through the additional electrode pairs.

More specifically, the paired electrode device (815) can have "n"-number of pairs of opposed electrodes, where "n" is a number or integer that is two or greater, such as 2, 3, 4,...or more. For such an electrode configuration, the paired electrode interface port (814) can have a like number of electrical connections for each of the "n"-number of pairs. The selection of the electrode pairs can be electrical, mechanical, or any combination thereof. For example, an electrical circuit such as the electrode pair selector (845) could be operably coupled to the paired electrode interface port (814) to select any desired combination of paired electrodes. This selector (845) can be operator selected, an automatic selection, a pre-set selection, or pre-programmed selection. Selection can also occur anywhere, for example, by having the electrode pair selector (845) in the switching matrix router (800) or in the surgical device such as paired electrode device (815). This electrode pair selector (845) could, in one embodiment, be a circuit that can recognize a surgical device such as the paired electrode device (815) and select an appropriate combination of pairs of electrodes for that device. The electrode pair selector (845) can be located, for example, anywhere between the electrodes and the power supply (801), including, but not limited to, on the handpiece, the connector, and the box for the router. The electrode pair and polarity selector (845) can also be mechanical, electrical or electromechanical such as a switch, a connector, or a jumper. For example, the electrode pair selector (845) could be as simple as an electrical connector on the ends of a plurality of electrode wires extending from the paired electrode device (815) and electrode selection can be the order in which they are plugged into a plurality of mating I/O connectors in the electrode interface port (814). Thus, for example, simply unplugging one combination of the electrode wires from the fixed connectors in the paired electrode interface port (814), and re-plugging different electrode wires into different connectors in the paired electrode interface port (814) can select new electrode pair combinations. Other combinations of four wires and four connectors can be selected for different electrode configurations for the electrode pair selector (845), however the selector (845) is not limited to that particular embodiment.

A module or paired electrode frequency switching device (830) can be electrically connected between the energy source (801) and the paired electrode interface port (814), and can operate with any connected embodiment of the paired electrode device (815) described above. When the paired electrode device (815) is connected to the paired electrode interface port (814), and energy is supplied from the energy source (801), the frequency switching module (830) may rapidly alternate or switch delivery of energy such as bipolar RF energy back and forth between any selected combination of electrode pairs and polarities at a switching frequency. With a paired electrode device (815) shown having two pairs of opposed electrodes, the alternating delivery of energy at a switching frequency can be timed to energize only one electrode pair at a time. As the electrode pairs are switched, the switching from the first pair to the second pair may be about instantaneous or near instantaneous. Alternately, by way of example, a slight pause or delay in the delivery of energy to the electrodes could occur slightly before the electrode pair switch, and/or during the switch, and/or slightly after the electrode pair switch. This delay between switching, for example, could be about 150 µseconds. Alternately, by way of example, whenever the paired electrode frequency switching module (830) is actuated or energy is being delivered, delivery of RF energy can be ensured to at least one pair of the selected electrode pairs such as pairs (816, 817) and (818, 819). In another alternate embodiment, the paired electrode frequency switching module (830) can prevent simultaneous power delivery to all electrode pairs.

The frequency switching matrix router (800) and/or switching module (830) can contain internal circuitry and logic to meet the switching needs of any embodiment described, or switching can be driven or controlled by an external device such as, but not limited to, the ASU. An ASU port (809) is provided to deliver power or signal from the ASU to the frequency switching module (830) and/or paired electrode device (815).

The paired electrode frequency switching module (830) may provide any one or any combination of switching frequencies. The appropriate frequency for a paired electrode device (815) can be selected as a function of surgical device and/or system parameters. These surgical device parameters can include, but are not limited to: spacing of the electrode pairs (816, 817) and (818, 819), the length of the electrodes, the RF power level with respect to tissue impedance, and the spring rate/compression force of the jaws holding the electrode pairs clamped on tissue. Selection of the appropriate frequency can be accomplished in a variety of ways through a frequency selector (818), which can be a manual switch such as a dial switch, a dip switch, jumpers, or a paired electrode device (815) recognition circuit using logic and/or surgical device recognition within the switching matrix router (800) or ASU. For example, the frequency selector (825) can identify the paired electrode device (815) and can select a switching frequency for that specific device. Such recognition could occur when the surgical device such as the paired electrode device (815) is electrically connected to the paired electrode interface port (814), and both device detection and frequency selection can be driven by the switching matrix router (800) or by the ASU. Alternately, the frequency selector (818) can be a portion of a surgical device such as paired electrode device (815).

Switching frequencies of the frequency switching module (830) can be between but are not limited to about 2 Hz and about 575 Hz. In another example, a frequency for a surgical device can range between 10 Hz to about 376 Hz. Alternately, in yet another embodiment, the frequency or a frequency range can be any selected from TABLE 1.

**TABLE 1**

| | Switching Frequency Selections (Hz) | |
|---|---|---|
| Selection No. | Nominal frequency | Frequency Range |
| 1 | 523 | 471-575 |
| 2 | 467 | 421-513 |
| 3 | 417 | 376-456 |
| 4 | 367 | 331-403 |
| 5 | 337 | 304-370 |
| 6 | 267 | 241-293 |
| 7 | 213 | 192-234 |
| 8 | 157 | 142-173 |
| 9 | 113 | 102-124 |
| 10 | 77 | 70-84 |
| 11 | 53 | 48-58 |
| 12 | 43 | 39-47 |
| 13 | 33 | 30-36 |
| 14 | 27 | 25-29 |
| 15 | 21 | 19-23 |
| 16 | 11 | 10-12 |
| 17 | 5 | 1-9 |

In addition to controlling and selecting the frequency, the paired electrode frequency shifting device (830) can be used to control the duty cycle for at least one of the pairs of the opposed electrodes. In an example, the paired electrode frequency shifting device (830) can alternate the energy between the respective pairs of electrodes at a duty cycle of less than 100% for each pair of electrodes.

Alternately, from a system level, the paired electrode frequency switching device (830) can be placed at any point between the energy source (801), and the paired electrodes such as the first pair of opposed electrodes (816, 817) and the second pair of opposed electrodes (818, 819).

While the matrix router and surgical device have been described as a utilizing bipolar RF energy, other energy sources may be utilized such as microwave energy. The surgical device can have n-number of paired (for bipolar application) sets of ablation energy delivery surfaces (such as electrodes when, e.g., RF energy is used) where n is greater than 2, and each electrode pair or active surface can cauterize tissue clamped therebetween or placed thereupon with any of the energies described above. In an embodiment, the alternate frequency switching matrix router can include a switching module that can alternate delivery of any energy to the n-numbered paired energy delivery surfaces of the alternate surgical device.

The RF energy monitored impedance delivered to the electrode pairs is preferably based, at least in part, on the monitored impedance of the tissue to be ablated as it is held between the jaws of the instrument. To this end, the controller preferably monitors or senses voltage and/or currents associated therewith, calculating or deriving the impedance of the tissue between the electrodes of at least one of the pairs of opposed electrodes and preferably between at least two of the pairs of opposed electrodes. The ablation may continue until the calculated impedance indicates that the lesion or ablation line is transmural (or fully through the tissue thickness).

With reference to FIGS. 2-4, an electrosurgical instrument 12 according to the present invention having two pairs of opposed electrodes is shown. The proximal jaw 20 has a first electrode 28 and a second electrode 30 in a proximal insulator 32 that extends along the length and across the width of the jaw. The electrodes 28, 30 are preferably centered laterally on the insulator 32 about the medial plane of the jaw and spaced apart a distance 34 of from about 0.7 mm to 4 mm, the distance being a factor of the thickness of the tissue to be ablated. Other factors influencing the distance 34 between the electrodes include the type of tissue (e.g. cardiac, skeletal muscle or smooth muscle, such as the small bowel or uterus wall), the frequency of energy source switching, the desired duration of ablation, and the desired width of ablation. If the tissue to be ablated is about 5 mm thick (uncompressed), which is typical of cardiac tissue, the preferred electrode spacing is approximately 1.0 mm. As the spacing of the electrodes is increased, it is preferred that the insulator surface be convex so that the desired increased pressure on the tissue between the electrodes is achieved. With two pairs of opposed electrodes, the crown radius of the insulator of a preferred embodiment is about 4.5 mm , and its face width is about 5 mm. These dimensions are illustrative only, and other dimensions may be used without departing from the present invention in its broader respects.

The distal jaw 22 is configured similarly to the proximal jaw 20 and has a third electrode 36 preferably directly opposite to first electrode 28 and a fourth electrode 38 preferably directly opposite to second electrode 30. Electrodes 36, 38 are mounted in a distal insulator 40 with an electrode spacing 42, preferably matching the spacing in the proximal jaw so that the electrodes in each pair are in opposed relation. Electrodes 28, 30, 36, 38 are preferably identical in size and shape and preferably have a beryllium-copper base with a nickel/gold plating covering all exposed surfaces. The electrodes have a preferred electrode width 44 of about 0.3 mm (.012 inches) and extend from the surface of the insulator over the length of the ablation surface from about 0.000 mm to about 0.15 mm. Other widths and projections may also be used.

With reference to FIGS. 5-7, the four electrodes 28, 30, 36, 38 can form parallel dual electrode arrays that can be pulsed. That is, electrodes 28 and 36 define one ablation energy source in the form of one pair of opposed electrodes having opposite polarity and electrodes 30 and 38 define another energy source in the form of a second pair of opposed electrodes that have opposite polarity. Although in the drawings the electrodes on each jaw are indicated as being of the same polarity, the electrodes on each jaw may be of the same or opposite polarity without departing from the invention. When energized by the RF generator, electrical current flows between the electrodes, creating an energy field in the form of current flow or flux between the electrodes that generally, for purposes of illustration, is shown in Figures 5 and 6. As can be seen there, the current density is highest at the electrode surfaces, i.e. at the interface between the electrodes and the tissue, which, prior to the present invention, would also be expected to be the area of primary tissue heating. However, in accordance with the present invention, the two energy sources or pairs of opposing electrodes can be pulsed by energizing the first electrode pair 28, 36 for a timed duration (FIG. 5), turning the first electrode pair 28, 36 off and energizing the second electrode pair 30, 38 for the another, preferably the same, timed duration (FIG. 6), turning the second electrode pair 30, 38 off and repeating the pulsing cycle (alternately energizing and de-energizing the first electrode pair 28, 36 and the second pair 30, 38), thus creating an area of overlapping current flux 44 (FIG. 7). The overlapping area of current flux is thus subjected to a substantially continuous current flow, while the other tissue, including tissue at the electrode interface, experiences only intermittent current flow. As a result, a zone of primary tissue heating is created in the area of overlap, which is spaced from the area of highest current density.

The cycling of the electrodes is preferably repeated until the ablation of the tissue is transmural. Thus, to achieve a transmural ablation line, the opposing pairs of electrodes are cycled in a series of on-off first pair, on-off second pair cycles until ablation is complete. It has been determined that transmural ablation is achieved when the temperature of the tissue reaches approximately 50°C. Thus, one or more temperature sensors may be associated with the jaws to provide an indication of the progress of ablation. The temperature sensor(s) may be located on one or both jaws between the electrodes forming part of each opposed pair or on the jaws outside of the electrodes to detect thermal spread, which is the lateral spread of heat from the area or zone of ablation into tissues outside the zone.

FIGS. 8-10 illustrate the proximal and distal jaws 20, 22 clamped on tissue 48 of a tissue thickness 50. The clamping pressure is pressing the jaws 20, 22 into the tissue to create a jaw gap 52 that is typically less than the tissue thickness 50. The clamping pressure may be controlled by a tissue pressure system, such as a lost motion means, located in the handle of the electrosurgical device in a manner known as the prior art. Note that the tissue is compressed between two arcuate surfaces formed from the electrodes 28, 30, 36, 38 and the proximal insulator 32 and the distal insulator 40. These arcuate surfaces preferably apply the highest tissue pressure across the narrowest clamp gap 52 and the electrodes 28, 30, 36, 38 flank this high pressure region. The pressure exerted on the tissue to be ablated is preferably sufficient to reduce the moisture in the tissue held between the jaws. This pressure is proportional to the thickness of the tissue to be ablated, and is typically between about 68.94 and 158.56 KPa (10 and 23 psi) for typical cardiac tissue having an uncompressed thickness of about 5 mm, with a preferred clamping pressure of about 110.3 KPa (16 psi.) for cardiac tissue having a compressed thickness of about 2 mm.

As indicated above, by energizing the electrodes alternately, as shown in FIGS. 5-7, a zone of primary heating is created in tissue held therebetween where there is a continuous flux, that is where the fluxes overlap, as designated 44 in FIG. 7. A zone of primary heating results that is offset from the zones of highest current density, which is directly between the electrodes of each pair, as shown in FIGS. 5 and 6. The pulsing frequency is preferably between about 2 and 575 Hz, and more preferably between about 10 and 376 Hz, with a higher frequency being preferred for ablating thinner tissue structures and a lower frequency being preferred for thicker tissue structures. The alternate energizing or pulsing of the electrodes means that the electrodes energized only 50 percent of the time if the on-off cycles are identical. This half-duty cycle provides for balanced ablation and allows the electrode surfaces to be substantially cooler than if they were operated continuously. As a result, ablation 54 starts centrally in the tissue in the zone of primary heating, and gradually extends or expands outwardly toward the jaw surfaces, as shown in FIGS. 8-10. Because the ablation does not start at the electrode surface, the impedance at the electrode surface does not increase as rapidly as with a single pair of opposed electrodes, resulting in a more efficient ablation process. If desired, the duty-cycle could be something other than half-on, half-off, such as 60% on, 40% off, to achieve an ablation biased to one side or the other.

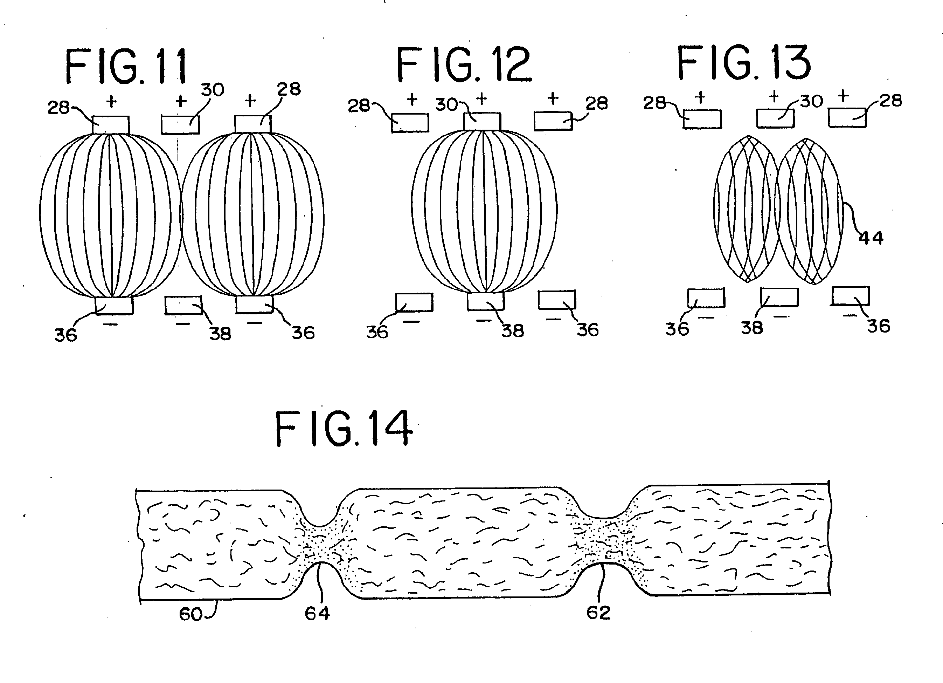

With reference to FIGS. 11-13, the present invention also contemplates using multiple energy sources, for example, three or more pairs of opposed electrodes, and alternately energizing the adjacent electrodes. With reference to FIGS. 11-13, for example, three pairs of opposed electrodes are schematically illustrated, with the outer two pairs being fired simultaneously (FIG. 11) and then being deactivated with the central electrode pair being fired (FIG. 12) to result in two zones of primary heating being created between the jaws corresponding to the two zones of overlapping current flux, both zones being spaced from the zones of highest current density that occurs directly between the electrodes of each pair and at the electrode-tissue interface. The number of electrode pairs utilized is selected based upon the desired width of the ablation line to be obtained. The number of electrode pairs may be more than three pairs, and may be an even or odd number of electrode pairs.

With reference to FIG. 14, there is seen a representation in cross-section of tissue 60 ablated with a device having a single pair of opposed electrodes and with a device having two pairs of opposed electrodes operated according to the methods described above. The ablation line cross-section 62 resulting from the use of two pairs of electrodes is wider than the ablation line cross-section 64 resulting from a similarly configured instrument having a single pair of opposed electrodes, resulting in an ablation line that is more visible to the surgeon. However, the ablation line 62 continues to be maintained within the width of the jaws and forms a relatively well-defined line of ablation with limited lateral extent and limited thermal spread to adjacent tissue. Thus, the device of the present invention provides for wider ablation lines that are well defined and do not spread beyond the width of the jaws. Ablations obtained by use of the device of the present invention are also wider at the core than those using a single pair of opposed electrodes, and have more of a barrel shape than an hour-glass shape as shown in the drawing.

While the present invention has been illustrated by description of several embodiments and while the illustrative embodiments have been described in considerable detail, it is not the intention of the applicant to restrict or in any way limit the scope of the appended claims to such detail. For example, while the invention has been described as using bipolar RF energy, other energy sources may be used, such as microwave energy. Additional advantages and modifications may readily appear to those skilled in the art.

## Claims

1. A tissue ablation system (10, 12) comprising:
an energy generator (14);
a plurality of ablation energy sources (28, 30, 36, 38) adapted to be positioned in proximity to tissue to be ablated, each energy source (i) being in operative communication with the generator, (ii) comprising at least two electrodes that together constitute a bi-polar electrode configuration and (iii) generating an energy field when energized, the energy field being in the form of electrical current flow between the electrodes,
wherein the energy sources are relatively positioned so that the energy fields of the sources partially overlap and wherein the tissue ablation system comprises a control system (800, 830) that is operatively associated with the generator and sources, **characterised in that** the control system is operable to alternately energize and de-energize the sources repeatedly so that, in use, tissue in the area of overlap is subjected to a substantially constant and continuous current flow while tissue at the electrode interface experiences only intermittent current flow.

2. The system of claim 1 including a monitor operable to monitor ablation of the tissue.

3. The system of claim 1 or 2 including a pair of opposed, relatively movable surfaces (20, 22) adapted to apply pressure to the tissue during ablation thereof.

4. The system of claim 3; wherein the opposed surfaces (20, 22) are adapted to apply pressure sufficient to reduce the moisture content of the tissue during ablation.

5. The system of claim 4, wherein the opposed surfaces (20, 22) are provided by proximal and distal jaws and the system further comprises a tissue pressure system that is operable to control the clamping pressure applied by the proximal and distal jaws.

6. The system of any one of claims 3 to 5, in which the opposed, relatively movable surfaces (20, 22) are adapted to apply pressure between about 68.94 and 158.56 kPa (10 and 23 psi) to the tissue to be ablated.

7. The system of claim 6 in which the opposed, relatively movable surfaces (20, 22) are adapted to apply pressure of about 110.3 kPa (about 16 psi).

8. The system of any one of claims 3 to 7, wherein the energy sources (28, 30, 36, 38) are carried on at least one of the opposed surfaces (20, 22).

9. The system of any one of the preceding claims, wherein each energy source comprises a RF energy source.

10. The system of any one of the preceding claims in which the electrodes are alternately energizable and de-energizable at a frequency of between about 2 and 575 Hz.

11. The system of claim 10 in which the electrodes are alternately energizable and de-energizable at a frequency of between about 10 and 376 Hz.

12. The system of any one of the preceding claims further including an impedance monitor operable to monitor impedance between the electrodes of at least one of the energy sources.

13. The system of any one of claims 1 to 11 further including an impedance monitor operable to monitor impedance between at least two of the energy sources.

14. The system of any one of the preceding claims, wherein the plurality of ablation energy sources are operable to create a plurality of areas of overlapping energy fields.

15. The system of any one of the preceding claims, wherein the density of the energy field is less in the area of overlap than closer to the energy source.

16. The system of any one of the preceding claims further comprising a temperature sensor for sensing the temperature of tissue.

17. The system of claim 16 in which the temperature sensor can be disposed to sense the temperature of tissue between the energy sources.

18. The system of claim 16 in which the temperature sensor can be disposed to sense the temperature of tissue outside the energy sources.

19. The system of any one of the preceding claims, wherein the number of energy sources is at least three, one of the energy sources being positioned between at least two other energy sources, said at least two other energy sources being energizable substantially simultaneously, and said one of the energy sources being energizable and de-energizable alternately with other two energy sources.

## Patentansprüche

1. Gewebeablationssystem (10, 12), das Folgendes umfasst:
einen Energiegenerator (14);
mehrere Ablationsenergiequellen (28, 30, 36, 38), die dafür ausgelegt sind, in der Nähe eines abzutragenden Gewebes positioniert zu werden, wobei jede Energiequelle: (i) in einer Wirkverbindung mit dem Generator steht; (ii) mindestens zwei Elektroden umfasst, die zusammen eine bipolare Elektrodenkonfiguration bilden; und (iii) bei Beaufschlagung mit Strom ein Energiefeld erzeugt, wobei das Energiefeld die Form eines elektrischen Stromflusses zwischen den Elektroden aufweist,
wobei die Energiequellen so relativ positioniert sind, dass sich die Energiefelder der Quellen teilweise überlappen, und wobei das Gewebeablationssystem ein Steuerungssystem (800, 830) umfasst, das mit dem Generator und den Quellen wirkverbunden ist, **dadurch gekennzeichnet, dass** das Steuerungssystem dafür geeignet ist, die Quellen im Wechsel wiederholt mit Strom zu beaufschlagen und vom Strom zu trennen, so dass Gewebe im Überlappungsbereich einem im Wesentlichen konstanten und dauerhaften Stromfluss ausgesetzt wird, während Gewebe an der Elektrodenschnittstelle lediglich einem intermittierenden Stromfluss ausgesetzt wird.

2. System nach Anspruch 1, das einen Monitor enthält, der dafür geeignet ist, die Ablation des Gewebes zu überwachen.

3. System nach Anspruch 1 oder 2, das ein Paar gegenüberliegender, relativ beweglicher Flächen (20, 22) enthält, die dafür geeignet sind, während der Ablation des Gewebes Druck auf das Gewebe auszuüben.

4. System nach Anspruch 3, wobei die gegenüberliegenden Flächen (20, 22) dafür geeignet sind, einen Druck auszuüben, der ausreicht, um den Feuchtigkeitsgehalt des Gewebes während der Ablation zu verringern.

5. System nach Anspruch 4, wobei die gegenüberliegenden Flächen (20, 22) durch proximale und distale Backen gebildet werden und das System des Weiteren ein Gewebedrucksystem umfasst, das dazu dient, den Klemmdruck zu steuern, der durch die proximalen und distalen Backen ausgeübt wird.

6. System nach einem der Ansprüche 3 bis 5, wobei die gegenüberliegenden, relativ beweglichen Flächen (20, 22) dafür geeignet sind, einen Druck zwischen etwa 68,94 und 158,56 kPa (10 und 23 psi) auf das abzutragende Gewebe auszuüben.

7. System nach Anspruch 6, wobei die gegenüberliegenden, relativ beweglichen Flächen (20, 22) dafür geeignet sind, einen Druck von etwa 110,3 kPa (etwa 16 psi) auszuüben.

8. System nach einem der Ansprüche 3 bis 7, wobei die Energiequellen (28, 30, 36, 38) auf mindestens einer der gegenüberliegenden Flächen (20, 22) getragen werden.

9. System nach einem der vorangehenden Ansprüche, wobei jede Energiequelle eine HF-Energiequelle umfasst.

10. System nach einem der vorangehenden Ansprüche, wobei die Elektroden im Wechsel mit einer Frequenz zwischen etwa 2 und 575 Hz mit Strom beaufschlagt und vom Strom getrennt werden können.

11. System nach Anspruch 10, wobei die Elektroden im Wechsel mit einer Frequenz zwischen etwa 10 und 376 Hz mit Strom beaufschlagt und vom Strom getrennt werden können.

12. System nach einem der vorangehenden Ansprüche, das des Weiteren einen Impedanzmonitor enthält, der dazu dient, die Impedanz zwischen den Elektroden von mindestens einer der Energiequellen zu überwachen.

13. System nach einem der Ansprüche 1 bis 11, das des Weiteren einen Impedanzmonitor enthält, der dazu dient, die Impedanz zwischen mindestens zwei der Energiequellen zu überwachen.

14. System nach einem der vorangehenden Ansprüche, wobei die mehreren Ablationsenergiequellen in der Lage sind, mehrere Bereiche überlappender Energiefelder zu erzeugen.

15. System nach einem der vorangehenden Ansprüche, wobei die Dichte des Energiefeldes im Überlappungsbereich geringer ist als in einem näheren Abstand zur Energiequelle.

16. System nach einem der vorangehenden Ansprüche, das des Weiteren einen Temperatursensor zum Detektieren der Temperatur von Gewebe umfasst.

17. System nach Anspruch 16, wobei der Temperatursensor so angeordnet werden kann, dass er die Temperatur von Gewebe zwischen den Energiequellen detektiert.

18. System nach Anspruch 16, wobei der Temperatursensor so angeordnet werden kann, dass er die Temperatur von Gewebe außerhalb der Energiequellen detektiert.

19. System nach einem der vorangehenden Ansprüche, wobei die Anzahl von Energiequellen mindestens drei ist, wobei eine der Energiequellen zwischen mindestens zwei anderen Energiequellen angeordnet ist, wobei die mindestens zwei anderen Energiequellen im Wesentlichen gleichzeitig mit Strom beaufschlagt werden können und die eine der Energiequellen im Wechsel mit den beiden anderen Energiequellen mit Strom beaufschlagt und vom Strom getrennt werden kann.

## Revendications

1. Système d'ablation de tissu (10, 12),
comprenant :
un générateur d'énergie (14) ;
une pluralité de sources d'énergie d'ablation (28, 30, 36, 38) adaptées pour être positionnées à proximité du tissu à ablater, chaque source d'énergie (i) étant en communication opérationnelle avec le générateur, (ii) comprenant au moins deux électrodes qui constituent ensemble une configuration d'électrode bipolaire et (iii) générant un champ énergétique lorsqu'elles sont excitées, le champ énergétique se présentant sous la forme d'un flux de courant électrique entre les électrodes,
dans lequel les sources d'énergie sont positionnées de manière relative de sorte que les champs énergétiques des sources se chevauchent partiellement et dans lequel le système d'ablation de tissu comprend un système de commande (800, 830) qui est associé de manière opérationnelle avec le générateur et les sources, **caractérisé en ce que** le système de commande peut fonctionner pour activer et désactiver de manière alternée les sources de manière répétée de sorte qu'à l'usage, le tissu dans la zone de chevauchement est soumis à un flux de courant sensiblement constant et continu alors que le tissu au niveau de l'interface d'électrode ne subit que le flux de courant intermittent.

2. Système selon la revendication 1, comprenant un moniteur pouvant fonctionner pour surveiller l'ablation du tissu.

3. Système selon la revendication 1 ou 2, comprenant une paire de surfaces (20, 22) opposées relativement mobiles, adaptées pour appliquer une pression sur le tissu pendant son ablation.

4. Système selon la revendication 3, dans lequel les surfaces opposées (20, 22) sont adaptées pour appliquer la pression suffisante afin de réduire la teneur en humidité du tissu pendant l'ablation.

5. Système selon la revendication 4, dans lequel les surfaces opposées (20, 22) sont fournies par les mâchoires proximale et distale et le système comprend en outre un système de pression de tissu qui peut fonctionner pour commander la pression de serrage appliquée par les mâchoires proximale et distale.

6. Système selon l'une quelconque des revendications 3 à 5, dans lequel les surfaces opposées (20, 22) relativement mobiles sont adaptées pour appliquer une pression comprise entre environ 68,94 et 158,56 kPa (10 et 23 psi) sur le tissu à ablater.

7. Système selon la revendication 6, dans lequel les surfaces opposées (20, 22) relativement mobiles sont adaptées pour appliquer une pression d'environ 110,3 kPa (environ 16 psi).

8. Système selon l'une quelconque des revendications 3 à 7, dans lequel les sources d'énergie (28, 30, 36, 38) sont supportées sur au moins l'une des surfaces opposées (20, 22).

9. Système selon l'une quelconque des revendications précédentes, dans lequel chaque source d'énergie comprend une source d'énergie RF.

10. Système selon l'une quelconque des revendications précédentes, dans lequel les électrodes peuvent être activées et désactivées de manière alternée à une fréquence comprise entre environ 2 et 575 Hz.

11. Système selon la revendication 10, dans lequel les électrodes peuvent être activées et désactivées de manière alternée à une fréquence comprise entre environ 10 et 376 Hz.

12. Système selon l'une quelconque des revendications précédentes, comprenant en outre un moniteur d'impédance pouvant fonctionner pour surveiller l'impédance entre les électrodes d'au moins l'une des sources d'énergie.

13. Système selon l'une quelconque des revendications 1 à 11, comprenant en outre un moniteur d'impédance pouvant fonctionner pour surveiller l'impédance entre au moins deux des sources d'énergie.

14. Système selon l'une quelconque des revendications précédentes, dans lequel on peut actionner la pluralité de sources d'énergie d'ablation pour créer une pluralité de zones de champs énergétiques qui se chevauchent.

15. Système selon l'une quelconque des revendications précédentes, dans lequel la densité du champ énergétique est inférieure dans la zone de chevauchement qu'à proximité de la source d'énergie.

16. Système selon l'une quelconque des revendications précédentes, comprenant en outre un capteur de température pour détecter la température du tissu.

17. Système selon la revendication 16, dans lequel le capteur de température peut être disposé pour capter la température du tissu entre les sources d'énergie.

18. Système selon la revendication 16, dans lequel le capteur de température peut être disposé pour capter la température du tissu hors des sources d'énergie.

19. Système selon l'une quelconque des revendications précédentes, dans lequel le nombre de sources d'énergie est au moins de trois, l'une des sources d'énergie étant positionnée entre au moins deux autres sources d'énergie, lesdites au moins deux autres sources d'énergie pouvant être activées de manière sensiblement simultanée, et ladite une des sources d'énergie pouvant être activée et désactivée de manière alternée avec les deux autres sources d'énergie.
